# EUROPEAN PATENT SPECIFICATION

(11) **EP 2 590 692 B1**
(45) Date of publication and mention of the grant of the patent: **08.03.2017**
(21) Application number: 11748763.7
(22) Date of filing: 05.07.2011
(51) Int. Cl.: A61L 27/34, A61L 27/04

(54) **MULTILAYERED PROTECTIVE COATING FOR PROTECTING METALLIC SURFACES OF IMPLANT MATERIALS AND USE THEREOF**
MEHRSCHICHTE SCHUTZBESCHICHTUNG FÜR DEN SCHUTZ VON METALLOBERFLÄCHEN VON IMPLANTATMATERIALIEN UND IHRE VERWENDUNG
REVÊTEMENT DE PROTECTION MULTICOUCHE POUR LA PROTECTION DE SURFACES MÉTALLIQUES DE MATÉRIAUX D'IMPLANT ET SON UTILISATION

(30) Priority: 06.07.2010 PL 39175310
(43) Date of publication of application: 15.05.2013
(73) Proprietor: Uniwersytet Jagiellonski, 31007 Krakow (PL)
(72) Inventor: CIESLIK, Monika, 32-077 Smardzowice (PL); KOTARBA, Andrzej, 30-033 Krakow (PL); ENGVALL, Klas, 186 50 Vallentuna (SE); LINDSTRÖM, Annika, 113 23 Stockholm (SE)
(74) Representative: Padée, Grazyna
(86) International application number: PCT/PL2011/000069
(87) International publication number: WO 2012/005614

(56) References cited:
- WO-A1-2007/025059
- WO-A1-2007/075003
- Hassler C; Von Metzen R P; Ruther P; Stieglitz T: "Characterization of parylene C as an encapsulation material for implanted neural prostheses", Journal of Biomedical Materials Research - Part B Applied Biomaterials, vol. 93B 29 January 2010 (2010-01-29), pages 266-274, XP55009294, DOI: 10.1002/jbm.b.31584 Retrieved from the Internet: URL:http://onlinelibrary.wiley.com/store/1 0.1002/jbm.b.31584/asset/31584_ftp.pdf?v=1 &t=gtnynvpt&s=54ba982c20c6f354d2b127d0e0c3 3d08e48e4d51 [retrieved on 2011-10-12]

## Description

The invention provides a multilayered protective coating for protecting surfaces of metal implants and the application of thereof.

In the 21st century, the progress in medical sciences and in physico-chemical measurement methods, to evaluate the performance, has resulted in increased demands on the functionality of implants. The 2000-2010 decade was proclaimed by the Secretary General of the United Nations and the World Health Organization as the "bone and joint decade" - the issue of motor organ diseases is one of the most serious threats to the health of Earth's inhabitants in the approaching millennium (Geneve, 13.01.2000). Both in Poland and globally, a dynamic increase is observed on the market of biomaterials. In particular, the demands are growing for metal implants, which could effectively seize the functions of damaged bones and allow patients to function normally in everyday life. Moreover, demands for implant surgery concern not only elderly persons suffering from osteoporosis (statistical data envisage that in the whole world 6.3 million of osteoporotic fractures would occur in 2050, compared to only 1.6 million in 1950), but gradually more also younger persons who play sports actively. Statistical data indicates that the number of fractures (even of lower leg bones), requiring use of metal implants in the treatment grows continuously for this group of patients.

Surgical procedures connected with insertion a metal implant inside an organism are complicated and connected with the risk of implant rejection by the organism. After introducing a metal implant into the organism a series of complex processes takes place on the implant-tissue interface. Various proteins and even entire cells are deposited on the implant's surface. In the specific case of metal implants, problems with release of ions of heavy metals from the implant (e.g. for steel: iron, chromium, nickel; for titanium alloys: titanium, vanadium, aluminium; for cobalt alloys: cobalt, chromium, molybdenum) into the organism is of particular concern. This release is connected directly with corrosion processes. Conditions prevailing in the organism favour corrosion processes (physiological fluid conditions, the temperature of 36.6°C). Due to complexity of the problem, changeable conditions in various parts of the organism, different processes taking place on the implant's surface has not been investigated in detail. Kinetics of metal ion release from the implant's surface is relatively slow, but regarding the very long implant residence time in the organism (from several weeks to more than ten years) the amounts of metals transferred into the organism are of great importance for patient's health. Metal ions at a concentration exceeding acceptable levels (np. for iron - 4000 - 5000 mg/70 kg body weight, for chromium - <6 mg/70 kg body weight, for nickel - ∼1 mg/70 kg body weight), are detrimental for a human organism, developing various allergic reactions, and even possibly leading to neoplastic changes. It was one of the reasons for withdrawing steel implants from long-term applications by replacing them with implants made of titanium.

An alternative solution includes the use of a material safe for a human organism, while retaining low production costs of the implant. There are still literature references reporting on studies of steel surface modifications to protect it against aggressive attacks from the body fluids, which may result in corrosion. Initially, attempts to inhibit metal ion release processes from steel surfaces by the use of appropriate surface treatments (e.g. polishing and electro-polishing, passivation), was investigated (Corros. Sci. 48 (2006) 2120). Since such surface modification methods did not functioned satisfactory, research is conducted on application of protective coatings. Such coatings should provide the needed protection of the surface against aggressive attacks of the physiological fluids, and, on the other hand, preclude transfer of metal ions from the implant into the organism. In the scientific literature and patent databases numerous reports are found on extensive research still conducted on use of biocompatible ceramic and polymer coatings *(*J. Antimicrob. Chemoth. 51 (2003) 585*,* J. Appl. Polym. Sci. 112 (2009) 3677*).* Such coatings are mainly aimed at protecting the implant surface against abrasive wear, enhancing integration of an implant with the surrounding tissue (e.g. US Patent No. 5,480,438 concerns use of bioactive ceramic coatings enhancing osteointegration of the metal implant with a bone) and enhancing corrosion resistance of the surface of metal implants.

It should also be considered that known patents and literature reports do not pay attention to one of the most dangerous effects for the patient connected with implant insertion. The effect includes the release of metal ions from the implant's surface into the organism *(*Biomaterials 26 (2005) 11*)*. Amounts of metals released are variable and depend on the implantation site, which determines the environment. Although the problem was already identified in the eighties, no proper attention was paid to the problem, and it was mainly solved by substituting steel materials with new alloys forming surfaces more stable in the environment of the organism. Lately, an increased interest in the problems is noted. This is most likely connected with increased demands on implants and their wide employment. It applies generally to short-term orthopaedic implants used as stabilizers for immobilization of broken bones (wires, rods, screws, plates etc.). In this case, the most popular material is austenitic stainless steel. Average residence time in the organism is from several weeks to several months. Such a period is sufficient for release of metal ions to such exent that it definitely can result in a threat for patient's health, up to the development of metalosis. Typical levels released from the 1 cm² area of a metal implant for a week into 1 liter of a physiological fluid are: for iron - 0.38 µg, for chromium - 0.05 µg, for nickel - 1.57 µg, for titanium - < 5 µg, for aluminium - 3 µg, for vanadium - 5 µg (Mater. Sci. Eng. C 24 (2004) 745*).*

Attempts made to solve the problem are directed mainly to introducing protective coatings of ceramic nature. US Patent No. 5,037,438 discloses use of zirconium oxide as a protective coating to secure metallic implant materials from processes of metal ion release from the implant surface and from processes of implant surface abrasion. US Patent No. 5,211,833 states the use of continuous oxide coatings on the titanium implant surface. Laboratory studies concluded that it is more preferable to use polymer coatings as protective coatings, due to better adhesion to metal implants while retaining good mechanical properties. The important feature of polymer coatings is also their higher resistance to brittle cracking as compared to ceramic coatings *(*G. E. Wnek, G. L. Bowlin, Encyclopedia of Biomaterials and Biomedical Engineering 2-nd edition (2008) Vol. 4*).* Despite intensive research on the subject no satisfactory solution has been obtained to the problem of blocking (or substantial confinement) of release of ions.

One of the polymers biocompatible with surrounding tissue is parylene (A. Bioeng. Biomach. 11(2009) 19). Patent databases provide some examples which inform on the use of parylene coating in various applications. US Patent No. 6,776,792 discloses the use of the parylene coating for protecting stent surfaces from clot formation. There are reports concerning use of parylene coating on stents as a heparin-carrying coating. US Patent No. 6,558,315 discloses the use of parylene coating to protect a polymer implant surface from abrasive processes. Silane-parylene layers, which are applied directly to the surfaces of implants, such as stents, prostheses, and similar applications, are also known. Such layers, as stated in US Patent No. 2009/0285975 and EP Patent No. 0747069B1, are designed to separate an implant surface from the surrounding blood environment, which is needed in cases when the materials used are toxic or have thrombogenic properties (making appearance of clots possible). In scientific reports and patent databases (e.g. US20090270986) information on applications of ethylene-vinyl acetate (EVA) as a biocompatible polymer to protect implant surfaces from aggressive impact of body fluids on their surfaces can be found. It is often used in the context of being a carrier for medicines.

The international application WO2007075003 discloses an orthodontic wire, comprising: (i) a metal wire formed of a metal alloy material, (ii) a silver film applied to a surface of the metal wire, and (iii) a parylene film applied to a surface of the silver film to prevent the silver film from being discolored. A silan-based adhesion promoting film may be further provided between the silver film and the parylene film. WO2007025059 discloses an implantable medical device comprising: a metallic substrate comprising a surface; a base coating layer covalently bonded to the surface of the substrate, the base coating layer comprising a silane compound; and a parylene layer disposed on the base coating layer. A further layer comprising pEVA is disposed on the parylene layer. The coating protects the medical device from corrosion when implanted. C.Hassler and al. tested the adhesion of parylen C on Si₃N₄, polyimide, gold and platinum. The adhesion to Si₃N₄ and platinum was improved with Silane 174, whereas the adhesion to gold and polyimide could not be improved sufficiently with this method (Hassler C and al. Characterizaton of parylene C as an encapsulation material for implanted neural prostheses ", Journal of Biomedical Materials Research - Part B Applied Biomaterials, vol. 93B, 29 January 2010, p. 266-274*).*

In our research works, it was unexpectedly established that the use of a parylene layer on a properly prepared metal surface not only protects a implant surface from aggressive attacks from body fluids, but also is able to prevent the process of metal ion release into the organism. In this context, the immune response of the organism to the introduced foreign body in form of an implant is of great importance. A consequence of this reaction is overproduction of hydrogen peroxide around the implant. Concentration of the agent is so high that enzyme Catalase is not able to neutralize it. This results in increase of amounts of released ions even for a level of magnitude.

The invention provides the use of a multilayered protective coating consisting of a passive layer on the implant surface, an inner silane layer and an outer parylene layer for protecting metallic surfaces of implant materials from corrosion processes and release of heavy metal ions from the implant into the patient's organism.

The invention pertains also to a multilayered protective coating for protecting metallic surfaces of implant materials from corrosion processes and release of heavy metal ions from implant into the patient's organism comprising of at least three layers, where the inner layer is the silane layer, the outer layer is the parylene layer and the passive layer is located between the implant surface and said silane layer.

The coating according to the invention is preferably used for protecting implant steel surfaces.

The protective coating to be used according to the invention may comprise more than three layers. Besides the passive, silane and parylene layers, the coating could include an elastomer layer, this elastomer layer being located on the parylene layer.

The passive layer comprises preferably of a layer made by chemical passivation of metallic surfaces of implant materials, preferably by oxidation in a 20% HNO₃ solution for 10 to 120 minutes, at from 20 to 70°C. The silane layer preferably comprises a silicon-containing polymer characterized by good adhesion to metal substrates. Silanes from the broad family of: α-methacryloxypropyltrimetoxy-silane (A-174), β-(3,4-epoxy cyclohexyl)-ethyltrimethoxy silane (A-186), and γ-glycidoxy propyltrimethoxy silane, (A-187) could be used.

Preferably, layers are made from poly(p-xylilene) (parylene) and in case of biocompatibility, the function can be performed by poly(chloro-p-xylylene) or poly(p-xylylene) (parylene C and parylene N) from the group of parylenes.

The elastomer layer is preferably made of: the ethylene-vinyl acetate copolymer, a polyether-polyester copolymer, a polyester-urethane copolymer, a silicone elastomer.

Preferably, the implant surface to which the protective coating is applied to is characterized by a roughness Rₐ<0.05.

Preferably, the implant surface is degreased by applying an organic solvent and drying.

Preferably, the parylene layer is applied by Chemical Vapor Deposition (CVD) method.

Preferably, the parylene layer has thickness of at least 1 µm.

A process for the preparation of the multilayered protective coating of the invention is very simple. While the entire process includes several steps, the majority of them include dipping the starting material in consecutive solutions. The passive layer and silane layer provide good adhesion of the parylene coating to the substrate. The parylene layer is a proper protective layer against transfer of heavy metals ions from the metal implant surface into the organism. The elastomer layer, due to its plastic properties, prevents formation of cracks in the crystalline and rigid parylene layer.

Tests of metal ion release conducted in a laboratory incubator, in the physiological fluid environment simulating conditions prevailing within a human body, indicated that the application of the protective coating of the invention allows a restriction of the amount of heavy metals ions released from the steel surface by 50% - 95% in relation to a uncoated surface.

Coating was characterized by its phase composition, morphology and electron surface properties by: electrochemical impedance spectroscopy (EIS), scanning electron microscopy with X-ray microanalysis (SEM-EDX), confocal microscopy (MC), measurement of electron affinity by Kelvin probe (KP).

The invention is illustrated in detail by the working examples.

### Example 1

Samples of 316L cold-rolled and bright annealed (BA) steel were cut to chunks sized 20 x 20 mm² (thickness = 0.8 mm) and washed with acetone, ethanol, deionized water, and then etched according to ASTM A-380 standard (the solution of 15 to 25 % nitric acid + 1 to 8 % hydrofluoric acid, at 20 to 60 °C, for 5 to 60 minutes). The samples were then dip coated with a monolayer of silane A174 and air-dried (ca. 0.5 h). The steel samples with monolayers of silane thus prepared were coated by chemical vapor deposition with a layer (2 µm) of parylene N. At 150 °C, the dimer was evaporated from the solid phase to the gaseous phase. The gaseous phase was then heated to 650 °C. At this temperature, the dimer was decomposed into monomer particles. The monomer particles were transferred to an adjacent chamber, where the surface to be covered was exposed. There, at the room temperature and under the initial vacuum, spontaneous deposition of the monomer on the surface combined with polymerization occurred. The thickness of the deposited layer was controlled by the deposition time.

Inspection of the cross-sections of the steel samples with two-layer polymer coating (by confocal microscopy) did not reveal any defects neither across the material's volume, nor in the coating itself. The parylene coating was characterized by good adhesion to the steel substrate. Further, the results of inspection by scanning electron microscopy (SEM) showed that the coating had no visible defects and was heterogenous.

### Example 2

Heavy metal (iron, chromium, nickel) ion release tests were conducted on stainless steel 316L samples (passivated in 20% HNO₃ for 30 min. and at 50 °C ± 1°C) both coated and not coated with a double coating of silane A 174 + parylene N (coating thickness - 2 µm) to the artificial physiological Hanks solution (pH=7.4). Samples sized 20 x 20 mm² were analyzed. Prior to the experiment all samples were washed with 2 % RBS^{®} (an alkaline detergent containing anionic, cationic and nonionic surface active substances) for 10 min at 50 ± 1 °C to remove all organic compounds from sample surfaces. Then the samples were washed for 3 minutes in deionized water and air dried.

For securing credibility of measurements of amounts of metal ions released into the fluids, all containers used in *in vitro* tests were etched for 24 hours in 10 % HNO₃, followed by triple washing with deionized water to remove all metal-containing impurities.

Samples were charged into containers with 8 mL of Hanks solution and, to simulate processes proceeding inside a human organism, they were shaken in the incubator for 28 days (37 °C ± 0.1 °C). Reference containers with a fluid without steel samples were shaken in the same conditions as the sample containers. Then, the steel samples with the coating removed from the fluid were washed 3 times with deionized water and dried. Samples of the Hanks solution after the experiment were acidified with 100 µl of 60 % HNO₃ and analyzed on atomic absorption spectrometer (Perkin Elmer Model 3110). Analysis of the fluid revealed that, from the steel surface coated with the parylene coating, the following were transferred to the Hanks solution, respectively: 0.14 mg/L of iron, 0.0013 mg/L of chromium and 0.004 mg/L of nickel, which, as compared with uncoated samples, means drops in amounts of heavy metals ions transferred of 50 %, 94 %, and 55 %, respectively.

### Example 3

Heavy metal (iron, chromium, nickel) ion release tests were conducted on stainless steel 316L samples (passivated in 20 % HNO₃ for 30 minutes and at 50 °C ± 1°C) both coated and not coated with a double coating of silane A 174 + parylene C (the coating thickness - 8 µm) to the artificial physiological Hanks solution (pH=7.4). Samples sized 20 x 20 mm² were analyzed. The samples and containers for conducting metal ion release tests were prepared in analogy to Example 2.

Samples were charged into containers with 8 mL of Hanks solution and, to simulate processes proceeding inside a human organism, they were shaken in the incubator for 28 days (37 °C ± 0.1°C). Reference containers with a fluid without steel samples were shaken in the same conditions as the sample containers. Then, the steel samples removed from the fluid with the coating were washed 3 times with deionized water and dried. Samples of the Hanks solution after the experiment were acidified with 100 µl of 60 % HNO₃ and analyzed on inductively coupled plasma atomic emission ICP AES spectrometer (Perkin Elmer, Model Optima 2100). Analysis of the fluid revealed that, from the steel surface coated with parylene coating (parylene C) the following were transferred to the Hanks solution, respectively: 0.167 mg/L of iron, 0.002 mg/L of chromium, and 0.007 mg/L of nickel, which, as compared with uncoated samples, means drops in amounts of heavy metals ions transferred of more than 50 %.

### Example 4

Following Example 1, the steel samples were prepared and surface coated with a layer of silane A174 and parylene N (coating thickness 8 µm). Then they were coated by dip coating with a monolayer of silane A174 and air-dried (ca. 0.5h). The steel samples with monolayers of silane in analogy to Example 1, were coated with the parylene N coating (8 µm). The samples thus prepared were coated at 120 °C with the ethylene-vinyl acetate layer (3 µm) by dip coating and air-dried (ca. 0.5h). It was found that introduction of the additional elastomer layer enhanced mechanical properties of the protective layer, by elimination of stresses in the parylene layer. This is of particular importance in the case of contact with an aggressive corrosion environment (e.g. generation of hydrogen peroxide in immune reaction of the organism to a grafted implant), since, in such conditions in the absence of the elastomer layer, formation of cracks was observed in the parylene layer (microscopy inspection revealed cracks in the size range of 1 µm).

## Claims

1. Use of a multilayered protective coating comprising a passive layer on the implant surface, an inner silane layer and an outer parylene layer, for protecting metallic surfaces of implant materials from corrosion processes and release of heavy metal ions from implant into the patient's organism.

2. Use according to claim 1, **characterized in** the protective coating comprising additionally an elastomer layer, said elastomer layer being located outside said parylene layer.

3. Use according to claim 1, **characterized in that** the silane layer is made of a silane selected from the group consisting of: α- methacryloxypropyltrimetoxy-silane (silane A-174), β-(3,4-epoxy cyclohexyl)-ethyltrimethoxy silane (silane A-186) and γ-glycidoxy propyltrimethoxy silane (silane A-187).

4. Use according to claim 1, **characterized in that** the parylene layer is a layer of poly(chloro-p-xylylene) (parylene C) or poly(p-xylylene) (parylene N)

5. Use according to claim 1, **characterized in that** the passive layer is a layer prepared by chemical passivation of surfaces of implant materials.

6. Use according to claim 5, **characterized in that** said chemical passivation is conducted by oxidation in the 20% HNO₃ solution for 10 to 120 minutes at from 20 to 70°C.

7. Use according to claim 2, **characterized in that** the elastomer layer is made of a polymer selected from the group consisting of the ethylene-vinyl acetate copolymer, a polyether-polyester copolymer, a polyester-urethane copolymer, a silicone elastomer.

8. Use according to claim 1, **characterized in that** the implant surface the protective coating is applied on has roughness Rₐ<0,05.

9. Use according to claim 1, **characterized in that** the implant surface is degreased with an organic solvent and dried.

10. Use according to claim 1, **characterized in that** the parylene layer is applied by the chemical vapor deposition method.

11. Use according to claim 1, **characterized in that** the parylene layer has thickness of at least 1 µm.

12. Use according to claim 1, **characterized in that** the coating is used for protecting an implant steel surface.

13. A multilayered protective coating for protecting metallic surfaces of implant materials from corrosion processes and release of heavy metal ions from implant into the patient's organism, which comprises at least three layers, where the inner layer is the silane layer, the outer layer is the parylene layer and the passive layer is located between the implant surface and said silane layer.

14. The coating according to claim 13, **characterized in** additionally comprising an elastomer layer, said elastomer layer being located outside the parylene layer.

15. The coating according to claim 13, **characterized in that** the silane layer is a layer of: α-methacryloxypropyltrimetoxy-silane (silane A-174), β-(3,4-epoxy cyclohexyl)-ethyltrimethoxy silane (silane A-186), and γ-glycidoxy propyltrimethoxy silane (silane A-187).

16. The coating according to claim 13, **characterized in that** the parylene layer is a layer of poly(chloro-p-xylylene) (parylene C) or poly(p-xylylene) (parylene N).

17. The coating according to claim 13, **characterized in that** the passive layer is a layer prepared by chemical passivation of the surfaces of implant materials.

18. The coating according to claim 17, **characterized in that** the chemical passivation is conducted by oxidation in the 20% HNO₃ solution for 10 to 120 minutes, at from 20 to 70°C.

19. The coating according to claim 14, **characterized in that** the elastomer layer is made of a polymer selected from the group consisting of the ethylene-vinyl acetate copolymer, a polyether-polyester copolymer, a polyester-urethane copolymer, a silicone elastomer.

20. The coating according to claim 13, **characterized in that** the implant surface the protective coating is applied on has roughness Rₐ<0,05.

21. The coating according to claim 13 **characterized in that** the implant surface is degreased with an organic solvent and dried.

22. The coating according to claim 13, **characterized in that** the parylene layer is applied by the chemical vapor deposition method.

23. The coating according to claim 13, **characterized in that** the parylene layer has the thickness of at least 1 µm.

## Patentansprüche

1. Verwendung von mehrlagiger Schutzschicht, die eine passive Schicht auf der Implantatoberfläche, eine innere Silanschicht und eine äußere Parylenschicht aufweist, zum Schutz metallischer Oberflächen von Implantatmaterialien vor Korrosionsprozessen und Freisetzung von Schwermetallionen aus dem Implantat in den Organismus des Patienten.

2. Verwendung nach Anspruch 1, **dadurch gekennzeichnet, dass** die Schutzbeschichtung zusätzlich eine Elastomerschicht umfasst, wobei die Elastomerschicht außerhalb der Parylenschicht angeordnet ist.

3. Verwendung nach Anspruch 1, **dadurch gekennzeichnet, dass** die Silanschicht eine Schicht von: α-Methakryloxypropyltrimetoxy-Silane (Silane A-174), β-(3,4-Epoxycyclohexyl)-ethyltrimethoxy-Silane (Silane A-186) und γ-Glycidoxypropyltrimethoxy-Silane (Silane A-187) ist.

4. Verwendung nach Anspruch 1, **dadurch gekennzeichnet, dass** die Parylenschicht eine Schicht von Poly(chloro-p-xylylene) (Parylen C) oder Poly(p-xylylene) (Parylen N) ist.

5. Verwendung nach Anspruch 1, **dadurch gekennzeichnet, dass** die passive Schicht eine Schicht ist, die durch chemische Passivierung von Oberflächen von Implantatmaterialien hergestellt wird.

6. Verwendung nach Anspruch 5, **dadurch gekennzeichnet, dass** die chemische Passivierung durch Oxidation in der 20%-igen HNO-Lösung für 10 bis 120 Minuten bei 20 bis 70°C durchgeführt wird.

7. Verwendung nach Anspruch 2, **dadurch gekennzeichnet, dass** die Elastomerschicht aus einem Polymer, ausgewählt aus der Gruppe bestehend aus dem EthylenVinylacetat-Copolymer, einem Polyether-Polyester-Copolymer, einem Polyester-Urethan-Copolymer, einem Silikon-Elastomer, hergestellt ist.

8. Verwendung nach Anspruch 1, **dadurch gekennzeichnet, dass** die Implantatoberfläche, auf die die Schutzbeschichtung angewendet wird, hat eine Rauheit von Rₐ<0,05.

9. Verwendung nach Anspruch 1, **dadurch gekennzeichnet, dass** die Implantatoberfläche mit einem organischen Lösungsmittel entfettet und getrocknet wird.

10. Verwendung nach Anspruch 1, **dadurch gekennzeichnet, dass** die Parylenschicht anhand von dem chemischen Aufdampfverfahren aufgebracht wird.

11. Verwendung nach Anspruch 1, **dadurch gekennzeichnet, dass** die Parylenschicht eine Dicke von mindestens 1 µm aufweist.

12. Verwendung nach Anspruch 1, **dadurch gekennzeichnet, dass** Beschichtung zum Schutz einer Implantatstahloberfläche verwendet wird.

13. Mehrschichtige Schutzschicht zum Schutz metallischer Oberflächen von Implantatmaterialien vor Korrosionsprozessen und Freisetzung von Schwermetallionen aus dem Implantat in den Organismus des Patienten, die mindestens drei Schichten umfasst, wobei die innere Schicht die Silanschicht ist, die äußere Schicht Parylenschicht ist und die passive Schicht zwischen der Implantatoberfläche und der Silanschicht angeordnet ist.

14. Beschichtung nach Anspruch 13, **gekennzeichnet dadurch, dass** sie zusätzlich eine Elastomerschicht, die außerhalb der Parylenschicht angeordnet ist, enthält.

15. Beschichtung nach Anspruch 13, **gekennzeichnet dadurch, dass** die Silanschicht eine Schicht von: α-Methakryloxypropyltrimetoxy-Silane (Silane A-174), β-(3,4-Epoxycyclohexyl)-ethyltrimethoxy-Silane (Silane A-186) und γ-Glycidoxypropyltrimethoxy-Silane (Silane A-187) ist.

16. Beschichtung nach Anspruch 13, **gekennzeichnet dadurch, dass** die Parylenschicht eine Schicht von Poly(chlor-p-xylylene) (Parylene C) oder Poly(p-xylylene) (Parylene N) ist.

17. Beschichtung nach Anspruch 13, **gekennzeichnet dadurch, dass** die passive Schicht eine Schicht ist, die durch chemische Passivierung der Oberflächen von Implantatmaterialien hergestellt wird.

18. Beschichtung nach Anspruch 13, **gekennzeichnet dadurch, dass** die chemische Passivierung durch Oxidation in der 20%-igen HNO-Lösung für 10 bis 120 Minuten bei 20 bis 70°C durchgeführt wird.

19. Beschichtung nach Anspruch 14, **gekennzeichnet dadurch, dass** die Elastomerschicht aus einem Polymer, ausgewählt aus der Gruppe bestehend aus dem EthylenVinylacetat-Copolymer, einem Polyether-Polyester-Copolymer, einem Polyester-Urethan-Copolymer, einem Silikon-Elastomer, hergestellt ist.

20. Beschichtung nach Anspruch 13, **gekennzeichnet dadurch, dass** die Implantatoberfläche, auf die die Schutzschicht aufgetragen wird, eine Rauhigkeit Rₐ<0,05 aufweist.

21. Beschichtung nach Anspruch 13, **gekennzeichnet dadurch, dass** die Implantatoberfläche mit einem organischen Lösungsmittel entfettet und getrocknet wird.

22. Beschichtung nach Anspruch nach Anspruch 13, **gekennzeichnet dadurch, dass** die Parylenschicht anhand von dem chemischen Aufdampfverfahren aufgebracht wird.

23. Beschichtung nach Anspruch 13, **dadurch gekennzeichnet, dass** die Parylenschicht mindestens die Dicke von 1 µm aufweist.

## Revendications

1. Utilisation d'un revêtement de protection multicouche comprenant une couche passive sur la surface de l'implant, une couche interne de silane et une couche externe de parylène pour la protection des surfaces métalliques des matériaux de l'implant contre les processus de corrosion et contre la libération d'ions de métaux lourds de l'implant dans l'organisme du patient.

2. Utilisation selon la revendication 1, **caractérisée en ce que** le revêtement de protection comprend en outre une couche élastomère, ladite couche élastomère étant située à l'extérieur de ladite couche de parylène.

3. Utilisation selon la revendication 1, **caractérisée en ce que** la couche de silane est constituée d'un silane choisi dans le groupe comprenant : α-méthacryloxypropyltrimétoxysilane (silane A-174), β-(3,4-époxy-cyclohexyl)-éthyltriméthoxysilane-(silane A-186) et γ-glycidoxy-propyltriméthoxysilane (silane A-187).

4. Utilisation selon la revendication 1, **caractérisée en ce que** la couche de parylène est une couche de poly(chloro-p-xylylène) (parylène C) ou de poly(p-xylylène) (parylène N).

5. Uilisation selon la revendication 1, **caractérisée en ce que** la couche passive est une couche préparée par une passivation chimique des surfaces des matériaux d'implant.

6. Utilisation selon la revendication 5, **caractérisée en ce que** ladite passivation chimique est effectuée par oxydation dans une solution de 20% de HNO₃ pendant 10 à 120 minutes à une température de 20 à 70°C.

7. Utilisation selon la revendication 2, **caractérisée en ce que** la couche élastomère est constituée d'un polymère choisi dans un groupe composé d'un copolymère d'éthylène-acétate de vinyle, d'un copolymère de polyéther-polyester, d'un copolymère de polyester-uréthane ou d'un élastomère de silicone.

8. Utilisation selon la revendication 1, **caractérisée en ce que** la surface de l'implant sur laquelle le revêtement de protection est appliqué a une rugosité de Rᵤₙₑ<0,05.

9. Utilisation selon la revendication 1, **caractérisée en ce que** la surface de l'implant est dégraissée avec un solvant organique et séchée.

10. Utilisation selon la revendication 1, **caractérisée en ce que** la couche de parylène est appliquée par un procédé de dépôt chimique en phase vapeur.

11. Utilisation selon la revendication 1, **caractérisée en ce que** la couche de parylène a une épaisseur au moins égale à 1 µm.

12. Utilisation selon la revendication 1, **caractérisée en ce que** le revêtement est utilisé pour protéger la surface acier de l'implant.

13. Un revêtement protecteur multicouches pour protéger les surfaces métalliques des matériaux de l'implant contre les processus de corrosion et contre la libération d'ions de métaux lourds dans l'organisme du patient, comprenant au moins trois couches, où la couche interne est une couche de silane, la couche externe une couche de parylène et où la couche passive est située entre la surface de l'implant et ladite couche de silane.

14. Le revêtement selon la revendication 13, **caractérisé en ce qu'**il comprend en outre une couche élastomère, ladite couche élastomère étant située à l'extérieur de ladite couche de parylène.

15. Le revêtement selon la revendication 13, **caractérisé en ce que** la couche de silane est une couche de α-méthacryloxypropyltrimétoxy-silane (silane A-174), β-(3,4-époxy-cyclohexyl)-éthyltriméthoxysilane-(silane A-186) et γ-glycidoxy-propyltriméthoxysilane (silane A-187).

16. Le revêtement selon la revendication 13, **caractérisé en ce que** la couche de parylène est une couche de poly(chloro-p-xylylène) (parylène C) ou de poly(p-xylylène) (parylène N).

17. Le revêtement selon la revendication 13, **caractérisé en ce que** la couche passive est une couche préparée par une passivation chimique des surfaces des matériaux de l'implant.

18. Le revêtement selon la revendication 17, **caractérisé en ce que** la passivation chimique est effectuée par oxydation dans une solution de 20% de HNO₃ pendant 10 à 120 minutes à une température de 20 à 70°C.

19. Le revêtement selon la revendication 14, **caractérisé en ce que** la couche élastomère est constituée d'un polymère choisi dans un groupe composé d'un copolymère d'éthylène-acétate de vinyle, d'un copolymère de polyéther-polyester, d'un copolymère de polyester-uréthane ou d'un élastomère de silicone.

20. Le revêtement selon la revendication 13, **caractérisée en ce que** la surface de l'implant sur laquelle le revêtement de protection est appliqué a une rugosité de Rᵤₙₑ<0,05.

21. Le revêtement selon la revendication 13, **caractérisée en ce que** la surface de l'implant est dégraissée avec un solvant organique et séchée.

22. Le revêtement selon la revendication 13, **caractérisée en ce que** la couche de parylène est appliquée par un procédé de dépôt chimique en phase vapeur.

23. Le revêtement selon la revendication 13, **caractérisée en ce que** la couche de parylène a une épaisseur au moins égale à 1 µm.
